(19) Europäisches Patentamt
European Patent Office
Office européen des brevets

(11) Veröffentlichungsnummer: **0 019 763**
**B1**

# (12) EUROPÄISCHE PATENTSCHRIFT

(45) Veröffentlichungstag der Patentschrift: **01.08.84**

(51) Int. Cl.³: **C 07 J 5/00**, A 61 K 31/57 // C07J71/00

(21) Anmeldenummer: **80102494.4**

(22) Anmeldetag: **07.05.80**

(54) **Neue Kortikoide, ihre Herstellung und Verwendung.**

(30) Priorität: **18.05.79 DE 2920726**

(43) Veröffentlichungstag der Anmeldung: **10.12.80 Patentblatt 80/25**

(45) Bekanntmachung des Hinweises auf die Patenterteilung: **01.08.84 Patentblatt 84/31**

(84) Benannte Vertragsstaaten: **AT BE CH DE FR GB IT LI LU NL SE**

(56) Entgegenhaltungen:
**FR - A - 2 015 858**
**FR - M - 6 752**
**US - A - 3 232 839**
**US - A - 3 426 128**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Laurent, Henry, Dr., Glambecker Weg 21, D-1000 Berlin 28 (DE)**
Erfinder: **Wiechert, Rudolf, Prof., Petzower Strasse 8A, D-1000 Berlin 39 (DE)**
Erfinder: **Wendt, Hans, Dr., Ernst-Ring-Strasse 3, D-1000 Berlin 38 (DE)**
Erfinder: **Kapp, Joachim-Friedrich, Dr., Ludolfinger Weg 51, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 019 763 B1

**Beschreibung**

Die Erfindung betrifft den in den Patentansprüchen gekennzeichneten Gegenstand.

Die Kortikoide der allgemeinen Formel I gemäss Anspruch 1 können als Substituenten R ein Wasserstoffatom oder einen 1 bis 8 Kohlenstoffatome enthaltenden Acylrest tragen.

Unter einem Acylrest R soll vorzugsweise ein Formylrest, ein Alkanoylrest oder ein Benzoylrest verstanden werden. Geeignete Alkanoylreste sind beispielsweise: der Acetylrest, der Propionylrest, der Butyrylrest, der Isobutyrylrest, der Pentanoylrest, der Trimethylacetylrest oder der Hexanoylrest.

Anderseits soll unter einem Acylrest R vorzugsweise auch die Gruppierung

–CO–A–COOY,

worin

A eine Kohlenstoff-Kohlenstoffbindung oder eine Kohlenwasserstoffkette mit maximal 6 Kohlenstoffatomen und

Y ein Wasserstoffatom oder das Kation einer physiologisch unbedenklichen Base bedeuten, verstanden werden. Diese wasserlöslichen Kortikoide der allgemeinen Formel I sind 21-Monoester aliphatischer Dicarbonsäure, wie zum Beispiel der Oxalsäure, Malonsäure, Bernsteinsäure, Glutarsäure, Adipinsäure, Pimelinsäure, Maleinsäure, Fumarsäure usw. und deren Salze mit physiologisch unbedenklichen Basen, wie Alkali- oder Erdalkalimetallsalze (zum Beispiel Natriumsalz, Kaliumsalze oder Calziumsalze), Ammoniumsalze, Kupfer-II-salze, Methylglucaminsalze oder Aminosäuresalze.

Kortikoidhaltige pharmazeutische Präparate sind wegen ihres entzündungshemmenden Effekts heute bekanntlich für die Therapie, zum Beispiel in der inneren Medizin, in der Dermatologie aber auch in allen anderen klinischmedizinischen Bereichen, von überragender Bedeutung und nicht selten lebensrettend für den Patienten. Da aber alle systemisch wirksamen Kortikoide starke Nebenwirkungen (zum Beispiel Blutbildveränderungen, Zerstörung des lymphatischen Systems, Erhöhung der Infektionsanfälligkeit, Beeinflussung der Natriumretention, Atrophien der Milz, des Thymus der Nebenniere, des Körpergewichts usw.) verursachen, ist die Therapie mit kortikoidhaltigen Präparaten problematisch, zumal diese Nebenwirkungen bei der Dauertherapie mit Kortikoiden ständig vorhanden, oft am sogenannten Cushing-Syndrom des Patienten äusserlich erkennbar und nicht selten sogar lebensbedrohend sind.

In den Patentschriften US-A-3 232 839, FR-M-675 211, FR-A-2 015 858 und US-A-3 426 128 sind Kortikoide beschrieben, die bei topischer Anwendung eine ausgezeichnete inflammatorische Wirkung besitzen; die beispielsweise in diesen Patentschriften beschriebenen Verbindungen haben aber den Nachteil, dass sie auch noch sehr starke systemische Wirkungen aufweisen, die für die topische Applikation unerwünscht sind. Demgegenüber wurde gefunden, dass die erfindungsgemässen Verbindungen einen wesentlich günstigeren Dissozationsgrad zwischen erwünschter entzündungshemmender Wirksamkeit und unerwünschter Nebenwirkungen besitzen.

Die neuen Kortikoide der allgemeinen Formel I können in üblicher Weise zu Arzneimittelspezialitäten verarbeitet werden, indem man sie gegebenenfalls mit geeigneten Zusätzen, Trägersubstanzen, Lösungsvermittlern, Stabilisatoren und Geschmackskorrigentien in die gewünschten Applikationsformen, wie Salben, Cremes, Tabletten, Dragees, Kapseln, Lösungen usw. überführt.

Salben oder Cremes enthalten vorzugsweise 0,05 bis 1% des Wirkstoffes, Tabletten, Dragees oder Kapseln vorzugsweise 5 bis 200 mg des Wirkstoffes.

Ferner ist es möglich, pulverförmige Gemische, die beispielsweise 0,5 bis 20 mg Wirkstoff neben einem wasserlöslichen Träger wie Lactose enthalten, als Inhalationsmittel anzuwenden.

Zur Behandlung werden den Patienten je nach Schwere des Krankheitszustandes vorzugsweise 1 bis 1000 mg des Wirkstoffes pro Tag appliziert.

Die so erhaltenen Arzneimittelspezialitäten können zur Behandlung solcher Erkrankungen dienen bei denen üblicherweise eine Therapie mit Kortikoiden zweckmässig ist. Dies sind beispielsweise:

allergische Reaktionen, akut Schock und Kollaps, Status asthmaticus, zerebrales Ödem, ausgedehnte Verbrennungen, schwere Stoffwechselstörungen, akute schwere Dermatosen, Quincke Ödem, akute Infektionskrankheiten (Zusatztherapie), Polyarthritis rheumatica, akute und chronische Bindegewebeerkrankungen, allergische rheumatologische und dermatologische Erkrankungen, die auf eine orale Kortikoidtherapie ansprechen, kortikoidempfindliche Entzündungen der Mundschleimhaut, allergische Reaktionen, Lichen, entzündliche und allergische Erkrankungen des Auges, Iritis, Iridocyclitis, Konjuktivitis, Erkrankungen der vorderen Uvea, allergische und chronische Rhinitiden, Rhinitis vasomotorica, nicht eitrige Sinusitis, Heuschnupfen und Colitis ulcerosa.

Die Herstellung der neuen Kortikoide der allgemeinen Formel I erfolgt nach einem dem Fachmann wohlbekannten Verfahren (US-Patent 3 828 083).

Die für das erfindungsgemässe Verfahren verwendeten Ausgangsverbindungen der allgemeinen Formeln II und III können wie nachfolgend dargelegt, aus 6α-Chlor-11β,21-dihydroxy-16α-methyl-1,4-pregnadien-3,20-dion hergestellt werden, einer Verbindung, die nach dem bekannten Stand der Technik nur sehr schwierig hergestellt werden kann.

Auf dem nachfolgend geschilderten Wege ist diese Verbindung aber sehr einfach zugänglich.

a) Eine Lösung von 110 g 21-Acetoxy-16α-methyl-4-pregnen-3,20-dion in 550 ml Ethanol und 550 ml 1,2-Dimethoxyethan wird mit 11,2 g p-Toluolsulfonsäure und 116 g Orthoessigsäuretriethylester versetzt. Die Reaktionsmischung wird 50 Minuten auf 40 °C erhitzt, anschliessend auf 10 °C gekühlt, mit 12 ml Pyridin sowie 3 g Natriumacetat versetzt und in 3 Liter Eiswasser gegossen. Das Reaktionsprodukt wird mit Dichlormethan extrahiert, der Extrakt wird mit Wasser gewaschen, über Natriumsulfat getrocknet und im Vakuum eingedampft. Der Rückstand wird in 800 ml Aceton gelöst und die Lösung mit 540 ml Wasser, 17 ml Pyridin und 94 g Natriumacetat versetzt. Unter Rühren fügt man 106 g N-Chlorsuccinimid hinzu und rührt 30 Minuten bei 5 °C. Anschliessend wird eine Lösung von 37 g Natriumhydrogensulfit in 145 ml Wasser hinzugegeben und weitere 30 Minuten gerührt. Die Reaktionslösung wird in 15 Liter Eiswasser eingerührt, der ausgefällte Niederschlag wird abfiltriert, mit Wasser gewaschen und an der Luft getrocknet. Das Rohprodukt von 122 g wird an Kieselgel chromatographiert. Das gewünschte Produkt wird mit 10–22% Aceton-Dichlormethan eluiert und aus Aceton-Dichlormethan umkristallisiert. Ausbeute: 62,8 g 21-Acetoxy-6α-chlor-11β-hydroxy-16α-methyl-4-pregnen-3,20-dion. Schmelzpunkt: 209 °C. $[\alpha]_D^{25}$ = +176° (Pyridin). UV: $\varepsilon_{237}$ = 14 100 (Methanol).

b) 2,0 g 21-Acetoxy-6α-chlor-11β-hydroxy-16α-methyl-4-pregnen-3,20-dion löst man in 100 ml Dioxan, gibt 3 g 2,3-Dichlor-5,6-dicyan-1,4-benzochinon hinzu und erhitzt 24 Stunden zum Sieden. Nach dem Abkühlen wird vom Ungelösten abgesaugt, mit Dichlormethan gewaschen und im Vakuum eingeengt. Das Rohprodukt wird an Kieselgel chromatographiert. Mit 37–46% Aceton/Hexan erhält man, nach dem Umkristallisieren aus Aceton-Diisopropylether, 1,08 g 21-Acetoxy-6α-chlor-11β-hydroxy-16α-methyl-1,4-pregnadien-3,20-dion. Schmelzpunkt: 208,5 °C $[\alpha]_D^{25}$ = +90° (Chloroform). UV: $\varepsilon_{243}$ = 15 700 (Methanol).

c) 1,5 g 21-Acetoxy-6α-chlor-11β-hydroxy-16α-methyl-1,4-pregnadien-3,20-dion löst man in 42 ml Methanol, gibt 1,3 ml 70prozentige Perchlorsäure hinzu und lässt 20 Minuten bei Raumtemperatur stehen. Das Reaktionsgemisch wird in Eiswasser eingerührt, der ausgefallene Niederschlag abfiltriert, mit Wasser gewaschen und an der Luft getrocknet. Nach dem Umkristallisieren aus Aceton-Diisopropylether erhält man 1,02 g 6α-Chlor-11β, 21-dihydroxy-16α-methyl-1,4-pregnadien-3,20-dion. Schmelzpunkt: 128 °C. $[\alpha]_D^{25}$ = +55° (Chloroform). UV: $\varepsilon_{242}$ = 14 600 (Methanol).

Die einfache Synthese des 6α-Chlor-11β,21-dihydroxy-16α-methyl-1,4-pregnadien-3,20-dions bewirkt, dass die erfindungsgemässen Kortikoide der allgemeinen Formel I gemäss Anspruch 1, im Vergleich zu anderen hochwirksamen Kortikoiden mit relativ geringem Aufwand hergestellt werden können, was im Hinblick auf die hohen Wirkstoffkosten, mit denen kortikoidhaltige Arzneimittelspezialitäten belastet sind, nicht ohne Bedeutung ist.

Die nachfolgenden Beispiele dienen zur Erläuterung des erfindungsgemässen Verfahrens.

Beispiel 1

a) Eine Lösung von 14,0 g 21-Acetoxy-6α-chlor-11β-hydroxy-16α-methyl-1,4-pregnadien-3,20-dion in 29 ml Kollidin und 90 ml DMF wird mit 11 ml Methansulfonsäurechlorid, das 3% Schwefeldioxid enthält versetzt und bei Raumtemperatur gerührt. Nach 15 Minuten gibt man unter Eiskühlung 15 ml Wasser hinzu. Der entstandene Niederschlag wird abfiltriert, mit Wasser gewaschen und an der Luft getrocknet. Das Rohprodukt wird an Kieselgel chromatographiert. Mit 33–61% Essigester-Hexan erhält man 12,1 g 21-Acetoxy-6α-chlor-16α-methyl-1,4,9(11)-pregnatrien-3,20-dion. Eine Probe aus Essigester-Hexan umkristallisiert schmilzt bei 181 °C. $[\alpha]_D^{25}$ = +22° (Chloroform). UV: $\varepsilon_{236}$ = 15 600 (Methanol).

b) Eine Lösung von 12,0 g 21-Acetoxy-6α-chlor-16α-methyl-1,4,9(11)-pregnatrien-3,20-dion in 120 ml Dioxan wird nach Zugabe von 11,1 g N-Bromsuccinimid und 60 ml 10prozentiger Perchlorsäure bei Raumtemperatur gerührt und nach 60 Minuten in Eiswasser eingegossen. Das ausgefällte Produkt wird abfiltriert, mit Wasser gewaschen, an der Luft getrocknet und in 215 ml Ethanol gelöst. Nach Zugabe von 29 g Kaliumacetat wird die Lösung 8 Stunden unter Argon auf 60 °C erhitzt. Anschliessend wird im Vakuum auf ein Volumen von 60 ml eingeengt und in Eiswasser eingerührt. Das ausgefällte Produkt wird isoliert und an Kieselgel chromatographiert. Mit 8–9% Aceton-Hexan erhält man, nach dem Umkristallisieren aus Aceton-Diisoproylether, 5,2 g 21-Acetoxy-6α-chlor-9,11β-epoxy-16α-methyl-9β-pregna-1,4-dien-3,20-dion vom Schmelzpunkt 189 °C. $[\alpha]_D^{25}$ = +57° (Chloroform). UV: $\varepsilon_{247}$ = 15 600 (Methanol).

c) Zu 11 ml einer auf −60 °C gekühlten Lösung von Fluorwasserstoff in Pyridin (70prozentig) gibt man 800 mg 21-Acetoxy-6α-chlor-9,11β-epoxy-16α-methyl-9β-pregna-1,4-dien-3,20-dion in 3 ml Pyridin. Man lässt 15 Stunden bei Raumtemperatur stehen und rührt in 50 ml 10prozentige Ammoniumhydroxidlösung ein. Es wird mit Methylenchlorid extrahiert, mit verdünnter Salzsäure sowie mit Wasser gewaschen, getrocknet und im Vakuum eingeengt. Der Rückstand wird an Kieselgel chromatographiert. Mit 33–44% Essigester-Hexan erhält man 710 mg, die aus Essigester-Hexan umkristallisiert werden. Ausbeute: 600 mg 21-Acetoxy-6α-chlor-9α-fluor-11β-hydroxy-16α-methyl-1,4-pregnadien-3,20-dion. Schmelzpunkt: 240 °C. $[\alpha]_D^{25}$ = +82° (Chloroform). UV: $\varepsilon_{239}$ = 15 700 (Methanol).

Beispiel 2

Eine Lösung von 9,0 g 21-Acetoxy-6α-chlor-16α-methyl-1,4,9(11)-pregnatrien-3,20-dion in 340 ml Dioxan versetzt man mit 80 ml Wasser, 27 g N-Chlorsuccinimid und 27 ml 70prozentiger Perchlorsäure. Man lässt 30 Minuten bei Raumtemperatur reagieren und giesst in Eiswasser. Der ausgefällte Niederschlag wird abfiltriert, mit Wasser gewaschen, an der Luft getrocknet und an Kieselgel chromatographiert. Mit 35–53% Essigester-Hexan erhält man 7,23 g, die aus Essigester-

umkristallisiert, 5,33 g 21-Acetoxy-6α,9α-dichlor-11β-hydroxy-16α-methyl-1,4-pregnadien-3,20-dion ergeben. Schmelzpunkt: 238 °C. $[\alpha]_D^{25}$ = +110° (Chloroform). UV: $\varepsilon_{239}$ = 13 300 (Methanol).

Beispiel 3

1,90 g 21-Acetoxy-6α,9-Dichlor-11β-hydroxy-16α-methyl-1,4-pregnadien-3,20-dion werden in 53 ml Methanol gelöst. Man versetzt mit 1,7 ml 70prozentiger Perchlorsäure und lässt 20 Stunden bei Raumtemperatur stehen. Das Reaktionsgemisch wird in Eiswasser gegossen, das ausgefällte Produkt wird isoliert und an Kieselgel chromatographiert. Mit 37–52% Essigester-Dichlormethan erhält man, nach dem Umkristallisieren aus Aceton-Diisopropylether, 807 mg 6α,9α-Dichlor-11β,21-dihydroxy-16α-methyl-1,4-pregnadien-3,20-dion vom Schmelzpunkt 230 °C $[\alpha]_D^{25}$ = +99° (Chloroform). UV: $\varepsilon_{238}$ = 15 000 (Methanol).

Beispiel 4

Eine Lösung von 250 mg 6α,9-Dichlor-11β,21-dihydroxy-16α-methyl-1,4-pregnadien-3,20-dion in 5 ml Pyridin und 2,5 ml Buttersäureanhydrid wird 15 Stunden bei Raumtemperatur gerührt. Man versetzt mit Eiswasser und filtriert nach weiteren 3 Stunden den entstandenen Niederschlag ab. Das feuchte Rohprodukt wird in Dichlormethan aufgenommen, die Lösung wird mit Wasser gewaschen, über Natriumsulfat getrocknet und im Vakuum eingedampft. Der Rückstand wird aus Aceton-Diisopropylether umkristallisiert. Ausbeute: 166 mg 21-Butyryloxy-6α,9α-Dichlor-11β-hydroxy-16α-methyl-1,4-pregnadien-3,20-dion. Schmelzpunkt: 225 °C. $[\alpha]_D^{25}$ = 111° (Chloroform). UV: $\varepsilon_{238}$ = 15 500 (Methanol).

Beispiel 5

Eine Lösung von 250 mg 6α,9-Dichlor-11β,21-dihydroxy-16α-methyl-1,4-pregnadien-3,20-dion in 5 ml Pyridin wird nach Zugabe von 2,5 ml Valeriansäureanhydrid 15 Stunden bei Raumtemperatur gerührt. Man verfährt bei der Aufarbeitung wie im Beispiel 4 angegeben. Nach dem Umkristallisieren aus Aceton-Diisopropylether resultieren 165 mg 6α,9α-Dichlor-11β-hydroxy-16α-methyl-21-valeryloxy-1,4-pregnadien-3,20-dion vom Schmelzpunkt 218 °C. $[\alpha]_D^{25}$ = +113° (Chloroform). UV: $\varepsilon_{238}$ = 15 500 (Methanol).

Beispiel 6

Eine Lösung von 1,0 g 6α,9-Dichlor-11β,21-dihydroxy-16α-methyl-1,4-pregnadien-3,20-dion in 10 ml 1,2-Dichlorethan wird mit 2,5 ml Pivalinsäureanhydrid, 1,0 g Natriumhydroxid sowie 5 ml Wasser versetzt und 90 Minuten bei 30 °C gerührt. Nach Zugabe von 50 ml Dichlormethan wird die organische Phase abgetrennt, mit Wasser gewaschen, über Natriumsulfat getrocknet und im Vakuum eingeengt. Der Rückstand wird an Kieselgel chromatographiert. Mit 19–26% Essigester-Dichlormethan erhält man, nach dem Umkristallisieren aus Aceton-Diisopropylether, 245 mg 6α,9α-Dichlor-11β-hydroxy-16α-methyl-21-trimethylacetoxy-1,4-pregnadien-3,20-dion. Schmelzpunkt: 238 °C. $[\alpha]_D^{25}$ = +110° (Chloroform). UV: $\varepsilon_{238}$ = 15 500 (Methanol).

Beispiel 7

a) 2,5 g 6α-Chlor-11β,21-dihydroxy-16α-methyl-1,4-pregnadien-3,20-dion werden in 50 ml Pyridin und 25 ml Buttersäureanhydrid bei Raumtemperatur gerührt. Nach 15 Stunden wird in Eiswasser gegossen, der ausgefallene Niederschlag wird nach 3stündigem Stehenlassen abfiltriert, mit Wasser gewaschen und in Dichlormethan gelöst. Nach dem Trocknen und Einengen der Lösung wird der verbleibende Rückstand aus Aceton-Diisopropylether umkristallisiert. Man erhält 2,65 g 21-Butyryloxy-6α-chlor-11β-hydroxy-16α-methyl-1,4-pregnadien-3,20-dion vom Schmelzpunkt 192 °C. $[\alpha]_D^{25}$ = +82° (Chloroform). UV: $\varepsilon_{243}$ = 15 000 (Methanol).

b) 2,4 g 21-Butyryloxy-6α-chlor-11β-hydroxy-16α-methyl-1,4-pregnadien-3,20-dion werden, wie im Beispiel 1a beschrieben, in 21-Butyryloxy-6α-chlor-16α-methyl-1,4,9(11)-pregnatrien-3,20-dion überführt. Ausbeute: 2,1 g.

c) 2,1 g 21-Butyryloxy-6α-chlor-16α-methyl-1,4,9(11)-pregnatrien-3,20-dion werden, unter den im Beispiel 2 angegebenen Bedingungen, in 21-Butyryloxy-6α,9α-dichlor-11β-hydroxy-16α-methyl-1,4-pregnadien-3,20-dion überführt. Ausbeute: 1,23 g. Schmelzpunkt: 223 °C.

Beispiel 8

a) Eine Lösung von 2,25 g 6α-Chlor-11β,21-dihydroxy-16α-methyl-1,4-pregnadien-3,20-dion in 50 ml Pyridin und 25 ml Valeriansäureanhydrid wird 15 Stunden bei Raumtemperatur gerührt und danach in Eiswasser gegossen. Nach 4 Stunden wird der entstandene Niederschlag abfiltriert, mit Wasser gewaschen und in Dichlormethan gelöst. Die Lösung wird über Natriumsulfat getrocknet und im Vakuum eingedampft. Der Rückstand wird aus Aceton-Diisopropylether umkristallisiert. Ausbeute: 2,11 g 6α-Chlor-11β-hydroxy-16α-methyl-21-valeryloxy-1,4-pregnadien-3,20-dion. Schmelzpunkt: 229,5 °C $[\alpha]_D^{25}$ = +83° (Chloroform). UV: $\varepsilon_{243}$ = 15 600 (Methanol).

b) 2,0 g 6α-Chlor-11β-hydroxy-16α-methyl-21-valeryloxy-1,4-pregnadien-3,20-dion werden wie im Beispiel 1a beschrieben, in 6α-Chlor-16α-methyl-21-valeryloxy-1,4,9(11)-pregnatrien-3,20-dion überführt. Ausbeute: 1,74 g.

c) 1,74 g 6α-Chlor-16α-methyl-21-valeryloxy-1,4,9(11)-pregnatrien-3,20-dion werden, unter den im Beispiel 2 angegebenen Bedingungen, in 6α,9α-Dichlor-11β-hydroxy-16α-methyl-21-valeryloxy-1,4-pregnadien-3,20-dion überführt. Ausbeute: 1,02 g. Schmelzpunkt: 219 °C.

Beispiel 9

a) Eine Lösung von 2,0 g 6α-Chlor-11β,21-dihydroxy-16α-methyl-1,4-pregnadien-3,20-dion in 20 ml 1,2-Dichlorethan wird mit 5,2 ml Pivalinsäureanhydrid sowie 2,0 g Natriumhydroxid in 12 ml Wasser versetzt und 90 Minuten kräftig gerührt. Anschliessend wird mit Dichlormethan verdünnt, die organische Phase wird abgetrennt, mit

Wasser gewaschen, über Natriumsulfat getrocknet und im Vakuum eingeengt. Der Rückstand wird an Kieselgel chromatographiert mit 50% Essigester-Hexan erhält man, nach dem Umkristallisieren aus Aceton-Diisopropylether, 672 mg 6α-Chlor-11β-hydroxy-16α-methyl-21-trimethylacetoxy-1,4-pregnadien-3,20-dion. Schmelzpunkt: 189 °C. $[\alpha]_D^{25} = +78°$ (Chloroform). UV: $\varepsilon_{243} =$ 15 400 (Methanol).

b) 600 mg 6α-Chlor-11β-hydroxy-16α-methyl-21-trimethyl-acetoxy-1,4-pregnadien-3,20-dion werden, wie im Beispiel 1a beschrieben, in 6α-Chlor-16α-methyl-21-trimethylacetoxy-1,4,9(11)-pregnatrien-3,20-dion überführt. Ausbeute: 402 mg.

c) 402 mg 6α-Chlor-16α-methyl-21-trimethylacetoxy-1,4,9(11)-pregnatrien-3,20-dion werden, unter den im Beispiel 2 angegebenen Bedingungen, in 6α,9α-Dichlor-11β-hydroxy-16α-methyl-21-trimethylacetoxy-1,4-pregnadien-3,20-dion überführt. Ausbeute: 233 mg. Schmelzpunkt: 234 °C.

Beispiel 10

a) Man löst 2,3 g 6α-Chlor-11β,21-dihydroxy-16α-methyl-1,4-pregnadien-3,20-dion in einem Gemisch aus 55 ml Pyridin und 27 ml Capronsäureanhydrid und rührt 20 Stunden bei Raumtemperatur. Die Reaktionsmischung wird mit 10 ml Wasser versetzt, 3 Stunden auf dem Dampfbad erhitzt und in Eiswasser eingerührt. Der entstandene Niederschlag wird abfiltriert und in Dichlormethan gelöst. Die Lösung wird mit Wasser gewaschen über Natriumsulfat getrocknet und im Vakuum eingedampft. Der Rückstand wird aus Methanol umkristallisiert. Ausbeute: 2,0 g 6α-Chlor-21-hexanoyloxy-11β-hydroxy-16α-methyl-1,4-pregnadien-3,20-dion. Schmelzpunkt: 247 °C $[\alpha]_D^{25} = +118°$ (Pyridin). UV: $\varepsilon_{242} =$ 13 400 (Methanol).

b) 1,9 g 6α-Chlor-21-hexanoyloxy-11β-hydroxy-16α-methyl-1,4-pregnadien-3,20-dion werden, unter den im Beispiel 1 angegebenen Bedingungen, in 6α-Chlor-9α-fluor-21-hexanoyloxy-11β-hydroxy-16α-methyl-1,4-pregnadien-3,20-dion überführt. Ausbeute: 494 mg. Schmelzpunkt: 268 °C. $[\alpha]_D^{25} =$ +78° (Chloroform). UV: $\varepsilon_{239} =$ 15 600 (Methanol).

**Patentansprüche**

1. Kortikoide der allgemeinen Formel I

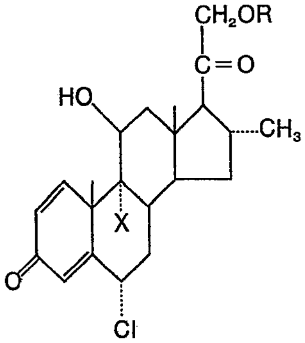

(I),

worin

X ein Fluoratom oder ein Chloratom und

R ein Wasserstoffatom oder einen 1 bis 8 Kohlenstoffatome enthaltender Acylrest darstellen.

2. 21-Acetoxy-6α-chlor-9α-fluor-11β-hydroxy-16α-methyl-1,4-pregnadien-3,20-dion.

3. 21-Acetoxy-6α,9α-dichlor-11β-hydroxy-16α-methyl-1,4-pregnadien-3,20-dion.

4. 6α,9α-Dichlor-11β,21-dihydroxy-16α-methyl-1,4-pregnadien-3,20-dion.

5. 21-Butyryloxy-6α,9α-dichlor-11β-hydroxy-16α-methyl-1,4-pregnadien,3,20-dion.

6. 6α,9α-Dichlor-11β-hydroxy-16α-methyl-21-valeryloxy-1,4-pregnadien-3,20-dion.

7. 6α,9α-Dichlor-11β-hydroxy-16α-methyl-21-trimethylacetoxy-1,4-pregnadien-3,20-dion.

8. 6α-Chlor-9α-fluor-21-hexanoyloxy-16α-methyl-1,4-pregnadien-3,20-dion.

9. Pharmazeutische Präparate, gekennzeichnet durch den Gehalt an ein oder zwei Kortikoiden gemäss Ansprüchen 1 bis 8.

10. Verfahren zur Herstellung von Kortikoiden der allgemeinen Formel I gemäss Anspruch 1, dadurch gekennzeichnet, dass man in an sich bekannter Weise

a) den Epoxydring eines Steroids der allgemeinen Formel II

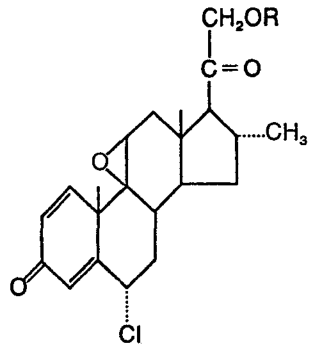

(II),

worin R die in Anspruch 1 genannte Bedeutung besitzt, mit Chlorwasserstoff oder Fluorwasserstoff öffnet oder

b) an die 9,11-Doppelbindung eines Steroids der allgemeinen Formel III

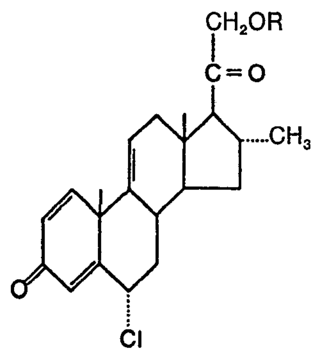

(III),

worin R die in Anspruch 1 genannte Bedeutung besitzt,

Chlorwasserstoff anlagert, und gegebenenfalls vorhandene Estergruppen verseift und/oder vorhandene 21-Hydroxygruppen verestert.

## Claims

1. Corticoids of the general formula I

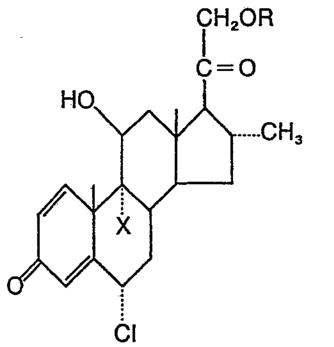

(I)

in which

X represents a fluorine atom or a chlorine atom and

R represents a hydrogen atom or an acyl radical containing from 1 to 8 carbon atoms.

2. 21-acetoxy-6α-chloro-9α-fluoro-11β-hydroxy-16α-methyl-1,4-pregnadien-3,20-dione.

3. 21-acetoxy-6α,9α-dichloro-11β-hydroxy-16α-methyl-1,4-pregnadien-3,20-dione.

4. 6α,9α-dichloro-11β,21-dihydroxy-16α-methyl-1,4-pregnadien-3,20-dione.

5. 21-butyryloxy-6α,9α-dichloro-11β-hydroxy-16α-methyl-1,4-pregnadien-3,20-dione.

6. 6α,9α-dichloro-11β-hydroxy-16α-methyl-21-valeryloxy-1,4-pregnadien-3,20-dione.

7. 6α,9α-dichloro-11β-hydroxy-16α-methyl-21-trimethylacetoxy-1,4-pregnadien-3,20-dione.

8. 6α-chloro-9α-fluoro-21-hexanoyloxy-16α-methyl-1,4-pregnadien-3,20-dione.

9. Pharmaceutical preparations, characterised in that they contain one or two corticoids according to claims 1 to 8.

10. Precess for the manufacture of corticoids of the general formula I according to claim 1, characterised in that in a manner known par se

a) the epoxide ring of a steroid of the general formula II

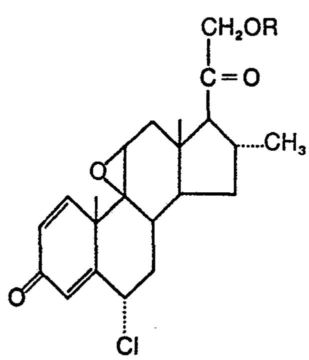

(II)

in which R has the meaning given in claim 1,

is opened by hydrogen chloride or hydrogen fluoride, or

b) hydrogen chloride is added on to the 9,11-double bond of a steroid of the general formula III

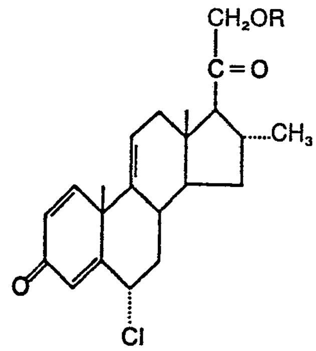

(III)

in which R has the meaning given in claim 1,

and optionally any ester groups present are hydrolysed and/or any 21-hydroxy groups present are esterified.

## Revendications

1. Corticostéroïdes répondant à la formule générale (I)

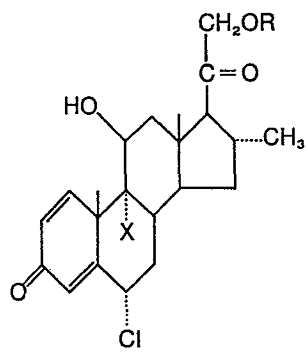

(I)

dans laquelle:

X représente un atome de fluor ou de chlore et

R représente un atome d'hydrogène ou un radical acyle contenant de 1 à 8 atomes de carbone.

2. Acétoxy-21 chloro-6α fluoro-9α-hydroxy-11β méthyl-16α prégnadiène-1,4 dione-3,20.

3. Acétoxy-21 dichloro-6α,9α hydroxy-11β méthyl-16α prégnadiène-1,4 dione-3,20.

4. Dichloro-6α,9α dihydroxy-11β,21 méthyl-16α prégnadiène-1,4 dione-3,20.

5. Butyryloxy-21 dichloro-6α,9α hydroxy-11β méthyl-16α prégnadiène-1,4 dione-3,20.

6. Dichloro-6α,9α hydroxy-11β méthyl-16α valéryloxy-21 prégnadiène-1,4 dione-3,20.

7. Dichloro-6α,9α hydroxy-11β méthyl-16α triméthyl-acétoxy-21 prégnadiène-1,4 dione-3,20.

8. Chloro-6α fluoro-9α hexanoyloxy-21 méthyl-16α prégnadiène-1,4 dione-3,20.

9. Médicaments caractérisés en ce qu'ils contiennent un ou deux corticostéroïdes selon l'une quelconque des revendications 1 à 8.

10. Procédé de préparation de corticostéroïdes de formule générale (I) selon la revendication 1, procédé caractérisé en ce que, en opérant de manière connue:

a) on ouvre le noyau époxy d'un stéroïde répondant à la formule générale (II)

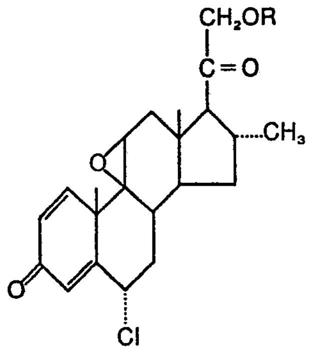

(II)

dans laquelle R a la signification donnée à la revendication 1, au moyen de chlorure d'hydrogène ou de fluorure d'hydrogène, ou

b) on fixe du chlorure d'hydrogène sur la double liaison, $\Delta^{9,11}$ d'un stéroïde répondant à la formule générale (III)

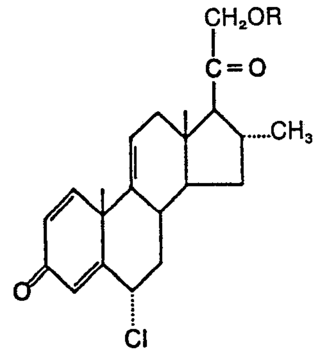

(III)

dans laquelle R a la signification donnée à la revendication 1,

et, éventuellement, on saponifie des radicaux d'esters s'il y en a et/ou on estérifie un éventuel radical hydroxy en 21.